(19)

Europäisches
Patentamt
European
Patent Office
Office européen
des brevets

(11) **EP 3 498 309 A1**

(12) **EUROPEAN PATENT APPLICATION**

(43) Date of publication:
**19.06.2019 Bulletin 2019/25**

(51) Int Cl.:
**A61L 27/26** (2006.01)    **A61L 27/52** (2006.01)
**A61L 27/54** (2006.01)    **A61L 27/60** (2006.01)

(21) Application number: **17306789.3**

(22) Date of filing: **15.12.2017**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD TN**

(71) Applicants:
 • **HRA Pharma Rare Diseases
 92320 Châtillon (FR)**
 • **Université de Cergy-Pontoise
 95000 Cergy (FR)**

(72) Inventors:
 • LARRETA-GARDE, Véronique
 95290 L'isle Adam (FR)
 • GOCZKOWSKI, Mathieu
 95320 Saint-Leu la Forêt (FR)
 • BEPPU, Marisa Masumi
 Campinas SP 13085-045 (BR)

(74) Representative: **Cabinet Becker et Associés
 25, rue Louis le Grand
 75002 Paris (FR)**

(54) **INTERPENETRATING POLYMER NETWORKS, AND THEIR METHODS OF MANUFACTURING**

(57)    The present invention relates to methods for obtaining a gel material comprising an interpenetrating polymer network (IPN) of fibrin and silk fibroin. It also relates to the gel materials obtainable by said methods. The present invention also relates to the use of said materials as materials for tissue engineering, in particular skin tissue engineering, as filling materials, and/or for drug release.

Figure 19

**EP 3 498 309 A1**

## Description

The present invention relates to methods for obtaining a gel material comprising an interpenetrating polymer network (IPN) of fibrin and silk fibroin. It also relates to the gel materials obtainable by said methods. The present invention also relates to the use of said materials as materials for tissue engineering, in particular skin tissue engineering, as filling materials, and/or for drug release.

## Technical Background

Fibrin gels, prepared from fibrinogen and thrombin, the key proteins involved in blood clotting, were among the first biomaterials used to prevent bleeding and promote wound healing. The unique polymerization mechanism of fibrin, which allows control of gelation times and network architecture by variation in reaction conditions, allows formation of a wide array of soft substrates under physiological conditions. In recent years, the application of fibrin gel in tissue engineering has also become more common. In comparison to the synthetic polymeric materials, fibrin gel presents many advantages, such as controllable degradation rate which matches those of tissue regeneration, nontoxic degradation products, and excellent biocompatibility.

However, the fibrin gels are soft materials, not self-supported when prepared at the physiologic concentration, and have poor rehydration rate. Those major drawbacks make fibrin hydrogels almost impossible to handle and to stock in dry state. Therefore, all commercially available fibrin-based products are kits to create the fibrin gel *in situ.*

To address these drawbacks, one strategy lies in the association of a fibrin gel with another polymer network, both being organized in an interpenetrating polymer network. Several systems have been developed that associate a fibrin gel with a synthetic polymer network. International patent application WO 2010/058132 describes a process for the preparation of a material comprising an interpenetrating polymer network of fibrin and polyethylene glycol. The synthetic network is cured by photopolymerization of polyethylene glycol, which bears reactive functions at its ends.

US patent 9,512,279 describes the preparation of an interpenetrating polymer network associating fibrin and a functionalized synthetic polymer, in the presence of a functionalized protein, such as bovine serum albumin. The reactive functions which are grafted on this protein enable the incorporation of the latter into the synthetic network during the photopolymerization step. This leads to a proteolytic degradation of the material.

However the photopolymerization step, which requires UV light and IRGACURE 2959® photoinitiator (1-[4-(2-Hydroxyethoxy)-phenyl]-2-hydroxy-2-methyl-1-propane-1-one), has drawbacks. UV light is known to be dangerous for cells, while photoactivation of the initiator releases free radicals. Hence, the conditions set for these methods have proven to be strongly cytotoxic.

There is thus a need for a safer gel material which can be obtained by a straightforward method.

Fibroin is an insoluble protein found in silk produced by certain animals such as spiders or the larvae of *Bombyx mori,* other moth genera such as *Antheraea, Cricula, Samia* and *Gonometa,* and numerous other insects. Most of the materials that associate silk fibroin and fibrin are hybrid materials, with a fibrin gel and a non-gelified silk fibroin. A material with both gelified fibrin and silk fibroin was reported by Pallotta et al. (Biomaterials, 2014, 35, 3678-3687). The method disclosed in this article employs a platelet-rich plasma as a fibrinogen and thrombin source, and the silk fibroin gelation is achieved through sonication of a silk fibroin solution. The structure of the material is not evidenced in this study.

The present invention is specifically intended to meet the above-mentioned need by providing a method for obtaining a gel material comprising an interpenetrating polymer network (IPN) of fibrin and silk fibroin, which gelation does not require toxic conditions and which can be advantageously used in the medical field.

## Summary of the invention

The present invention relates to a method for obtaining a gel material comprising an interpenetrating polymer network (IPN) of fibrin and silk fibroin, starting from fibrinogen, a silk fibroin solution and suitable gelation agents in aqueous solution.

In one embodiment, the invention provides a method for obtaining a gel material comprising an interpenetrating polymer network (IPN) of fibrin and silk fibroin, which method comprises contacting, in an aqueous solution, fibrinogen, a silk fibroin solution, a peroxidase, an oxidizing agent and thrombin, and incubating under conditions allowing the IPN of fibrin and silk fibroin to form.

In another embodiment, the method for obtaining a gel material comprising an interpenetrating polymer network (IPN) of fibrin and silk fibroin comprises the steps of:

(i) preparing a mixture comprising fibrinogen and silk fibroin in an aqueous solution;
(ii) adding thrombin to the mixture prepared in (i) and incubating under conditions allowing fibrinogen to convert into fibrin fibers that at least partially crosslink;

(iii) contacting the reaction mixture obtained in (ii) with an alcohol and incubating under conditions allowing the IPN to form.

The present invention also relates to the gel materials obtainable by said methods.

## Legends to the Figures

Abbreviations are specified in the Examples.

Figure 1 shows the *in situ* gelation kinetics of 1% Fb and 2% SF SNs and 2/1 SF/Fb IPN obtained through HRP/thrombin method followed by rheology (37°C, 1 Hz frequency, 1% strain).

Figure 2 shows the gelation kinetics in rheology of 1% Fb SN and 1% Fb Semi-IPN (37°C, 1 Hz frequency, 1% strain).

Figure 3 shows the gelation kinetics followed by rheology of 1% Fb semi-IPN turning into 2/1 SF/Fb IPN using different ethanol concentrations (30, 40 and 50% v/v). Analysis was performed ex situ, at 37°C, with 1 Hz frequency, 0.1% strain and 0.4 N normal force. Only storage moduli are presented.

Figure 4 shows a Western-Blot against fibrinogen of supernatant of chemically degraded fibrinogen (Fbg), 1% Fb and 2% SF SNs and 2/1 SF/Fb IPN obtained through ethanol/thrombin method.

Figure 5 shows the protein soluble extractable fraction of 1% Fb SN and semi-IPN and 2/1 SF/Fb IPN obtained through ethanol/thrombin method. Values are average $\pm$ SD of 8-9 samples (n=3) for 1% Fb SN and 1% Fb semi-IPN and 1 sample for 2/1 SF/Fb IPN.

Figure 6 shows CLSM (Obj. 40x, Scale bar 20 $\mu$m) images (Top) and SEM images (EHT 5 kV, VPSE mode at 100 Pa, Mag. 5000x, Scale bar 2 $\mu$m) of 1% Fb SN treated or not with 40% ethanol for 3 hours.

Figure 7 shows SEM images (EHT 5 kV, VPSE mode at 100 Pa, Mag 1000x) of 1 % Fb SN (A), 2% SF SN (B), and 2/1 SF/Fb IPN obtained through ethanol/thrombin method (C). Scale bar 10 $\mu$m.

Figure 8 shows the contraction of 1% Fb SN, 2% SF SN and 2/1 SF/Fb IPN obtained through ethanol/thrombin method, in the presence of fibroblasts (Fb-BJ). Results are average $\pm$ SD of 4 samples (n=1).

Figure 9 shows the viability of Fb-BJ cells seeded at the surface of the 2/1 SF/Fb IPN obtained through ethanol/thrombin method. Results are average $\pm$ SD of 7 to 12 fields obtained on 3 samples (n=1).

Figure 10 shows the macroscopic morphology of (left to right) 1% Fb SN, 2% SF SN and 2/1 SF/Fb IPN under visible light and UV 365 nm.

Figure 11 shows a Western-Blot against fibrinogen of supernatant of chemically degraded fibrinogen (Fbg), 1% Fb and 2% SF SNs and 2/1 SF/Fb IPN obtained through HRP-$H_2O_2$/thrombin method.

Figure 12 shows the protein soluble extractable fraction of 1% Fb SN, 2% SF SN and 2/1 SF/Fb IPN obtained through HRP-$H_2O_2$/thrombin method. Values are average $\pm$ SD of 9 samples (n=3).

Figure 13 shows SEM images (EHT 5 kV, VPSE mode at 100 Pa) of 1% Fb SN (A, B), 2% SF SN (C, D), and 2/1 SF/Fb IPN obtained through HRP-$H_2O_2$/thrombin method (E, F). For A, C and E: magnification 1000x, scale bar 10 $\mu$m. For B, D, F: magnification 5000x, scale bar 2 $\mu$m.

Figure 14 shows the degradation of 1% Fb SN, 2% SF SN and 2/1 SF/Fb IPN obtained through HRP-$H_2O_2$/thrombin method, by ThL 1 U/mL$_{mat}$. Values are average $\pm$ SD of 2 samples (n=1).

Figure 15 shows the degradation of 1% Fb SN, 2% SF SN and 2/1 SF/Fb IPN obtained through HRP-$H_2O_2$/thrombin method, by Trp 500 U/mL$_{mat}$. Values are average $\pm$ SD of 2 samples (n=1).

Figure 16 shows the contraction of 1% Fb SN, 2% SF SN and 2/1 SF/Fb IPN obtained through HRP-$H_2O_2$/thrombin method, in presence of fibroblasts (Fb-BJ). Results are average $\pm$ SD of 4 samples (n=1).

Figure 17 shows the viability of Fb-BJ cells seeded at the surface of the 2/1 SF/Fb IPN obtained through HRP-$H_2O_2$/thrombin method. Results are average $\pm$ SD of 7 to 12 fields obtained on 3 samples (n=1).

Figure 18 shows a particular embodiment of the "peroxidase" method.

Figure 19 shows a particular embodiment of the "alcohol" method.

## Detailed description of the invention

The present invention relates to methods for obtaining a gel material comprising an interpenetrating polymer network (IPN) of fibrin and silk fibroin, starting from fibrinogen, a silk fibroin solution and suitable gelation agents in aqueous solution.

In the context of the present invention, the expression "aqueous solution" refers to a liquid phase, which solvent is water and in which at least one solute is dissolved. The upper limit of the concentration of the at least one solute is given by its solubility, namely the maximum amount of the at least one solute that can be dissolved in a set amount of solvent at equilibrium. Preferably, the aqueous solution does not comprise a buffer.

In the context of the present invention, a solute is preferably a salt, or hydrates thereof.

Fibrinogen may be from any species, especially any mammal, preferably human, pork or bovine fibrinogen. Fibrinogen may be used in solution. It may be purified fibrinogen.

In an embodiment fibrinogen is obtained from serum or plasma. In a still preferred embodiment, the fibrinogen source does not comprise platelet.

Alternatively, and preferably, fibrinogen is recombinant fibrinogen. Recombinant fibrinogen may be obtained by production in a host cell, as widely described in the scientific and patent literature. Suitable examples of cells include insect cells, yeast cells, COS cells, BHK cells, NSO cells, Sp2/0 cells, CHO cells, PER.C6 cells and HEK293 cells. The host cell is preferably a mammalian cell. Production of recombinant fibrinogen can also be achieved in the milk of transgenic animals, such as sheep or cattle.

The silk fibroin is typically used in solution.

Native silk fibroins or recombinantly produced silk fibroin proteins may be used.

Silk fibroin can be obtained from spiders or the larvae of *Bombyx mori,* or other moth genera such as *Antheraea, Cricula, Samia* and *Gonometa,* and numerous other insects. In a preferred embodiment, the silk fibroin is obtained from *Bombyx mori* silkworm cocoons, e.g. using previously described procedures (e.g. Kim et al. Biomaterials, 2005, 26, 2775-2785).

Silk fibroin products are also commercially available, in lyophilized form or in solution. For instance, a silk fibroin solution is commercially available from ADVANCED BIOMATRIX.

Thrombin may be from any species, especially any mammal, preferably human, pork or bovine thrombin. Thrombin may be used in solution. In an embodiment, thrombin is obtained from serum or plasma. In another embodiment, thrombin is recombinant thrombin, which may be obtained by production in a host cell, as widely described in the scientific and patent literature. Suitable examples of cells include insect cells, yeast cells, COS cells, BHK cells, NSO cells, Sp2/0 cells, CHO cells, PER.C6 cells and HEK293 cells. The host cell is preferably a mammalian cell.

Advantageously, the gel material comprising an interpenetrating polymer network (IPN) of fibrin and silk fibroin obtainable by the methods according to the invention is homogeneous, easy to handle, stable over time, and display stable dimensions. Typically, said gel material may be stored for several weeks (e.g. 2 to 6 weeks) at a temperature comprised between 2 and 45 °C, e.g. 4°C, 20°C, or 37°C, without any structural and/or volume change.

The gel material of the invention comprises an interpenetrating polymer network (IPN) of fibrin and silk fibroin. No covalent bonds exist between the two networks.

The ratio of silk fibroin/fibrin comprised in the gel material is preferably 2/1.

The gel may be constituted of:

a) 0.2 to 2 % (*w/v*) of fibrin, preferably 0.3 to 1.2 % (*w/v*), more preferably 1 % (*w/v*);
b) 0.2 to 10 % (*w/v*) of silk fibroin, preferably 0.5 to 6 % (*w/v*), more preferably 2 % (*w/v*).

The gel material according to the invention displays enhanced mechanical properties compared to a gel comprising a fibrin network only.

At 37 °C and in a hydrated state, the gel material according to the invention may display a storage modulus G' comprised between 10 and 10000 Pa, for instance between 2000 and 6000 Pa, and a loss modulus G" comprised between 1 and 1000 Pa, for instance between 90 and 500 Pa.

The module G of a material subjected to a sinusoidal strain is written in the complex form:

$$G^* = G' + iG''$$

wherein i represents the imaginary part of a complex number.

In shear mode, the storage modulus of elasticity (G') is used to estimate the elasticity of the gel. The loss modulus or viscous modulus (G") characterizes the liquid phase of the gel. When G">G', the sample is regarded as liquid and when G'>G", the sample is considered as a gel. The gel time is considered as the time at which G' and G" moduli are equal.

The gel materials obtainable by a method according to the invention are advantageously biocompatible.

The gel materials obtainable by the methods of the invention are useful in

- wound dressing;
- hemostatic dressing;
- surgical dressing;
- coating for medical devices, e.g. stents, cardiac valves, catheters, vascular prosthetic filters;
- delivering active molecules, e.g. growth factors, antibiotics, bactericides, enzymes;
- providing support for eukaryotic cells culture.

The gel material may further comprise a drug. It is then useful for drug delivery, preferably sustained release. The gel material may particularly be in the form of a patch for transdermal delivery of a drug. The vaginal route may also be envisioned, e.g. for treating vaginal dryness. In a particular embodiment, the gel material may be used both for drug delivery and as a filling material.

Said drug may be e.g. a wound healing agent or a wound care agent, including anti-inflammatory agents, growth factors, agents which inhibit free radical formation, and bacteriostatic or bacteriocidal agents.

Non-limiting examples of the drug include lidocaine, a nonsteroidal anti-inflammatory drug, such as diclofenac, ibuprofen, ketoprofen, meloxicam or indomethacin, an antalgic such as morphine, codeine, tramadol or salicylic acid, an antiviral such as acyclovir or famcyclovir, a steroid, a corticosteroid such as methylprednisolone or prednisolone, a peptide such as insulin, or mixtures thereof.

In a particular embodiment, the drug may be a prostaglandin or a mixture of prostaglandins, e.g. for use in inducing labor in a pregnant woman, by placing the gel in the vagina of a pregnant woman, to induce labor.

In another embodiment, a bioactive agent can be covalently linked to the gel through a linker. The linker can be a cleavable linker or non-cleavable linker, depending on the application. As used herein, a "cleavable linker" refers to linkers that are capable of cleavage under various conditions.

In some embodiments, the gel is functionalized with a binding molecule that binds with a bioactive molecule. These binding molecules are also referred to as affinity molecules herein. The binding molecule can be bound covalently (directly or through a linker) or non-covalently to the matrix. The binding molecule can be selected such that it can bind to any part of bioactive molecule that is accessible.

Gels described herein can additionally include one or more additives.

The gels described herein can be used in tissue engineering and repair. For example, a gel described herein can be used to repair defects in cartilage, muscle, skin, tendon or ligament, bone, cornea. In another example, an implant can be coated with a gel before implanting. In yet another example, a degradable material can be coated with the gel to allow the degradable material to adhere to a tissue of interest. This can allow repairing of larger defect sizes by providing a support material that is coated with the gel thus allowing coverage of the larger defect size.

In another aspect, provided herein is a method of repairing a tissue defect in a subject in need thereof, the method comprising administering to the subject a gel described herein. As used herein, the term "tissue defect" refers to any medical abnormality of a tissue, including, but not limited to, a damaged tissue, a deficient tissue, a degraded tissue, a traumatized tissue. In fact, any abnormality of a tissue that can be repaired by a method of the invention is included in the term "tissue defect".

The methods for manufacturing the desired gel materials comprising an interpenetrating polymer network (IPN) of fibrin and silk fibroin are described in greater details below.

The "peroxidase" method:

In one embodiment, the invention provides a method for obtaining a gel material comprising an interpenetrating polymer network (IPN) of fibrin and silk fibroin, which method comprises contacting, in an aqueous solution, fibrinogen, a silk fibroin solution, a peroxidase, an oxidizing agent and thrombin, and incubating under conditions allowing the IPN of fibrin and silk fibroin to form.

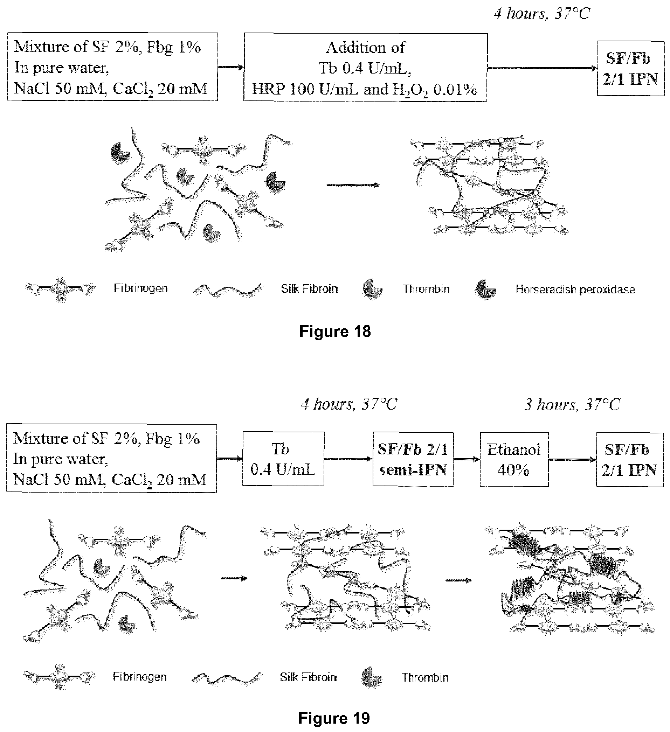

A particular embodiment of this method is shown on Figure 18.

The aqueous solution may comprise at least one salt, and preferably the aqueous solution comprises one salt and optionally further comprises a second salt.

More preferably, the aqueous solution comprises a divalent salt and optionally further comprises a second salt. In the present invention, a divalent salt results from the electroneutral association of one or several cations $X^{2+}$ with one or several anions $Y^{n-}$, n being an integer of 1 to 5, and X and Y being independently an atom or a group of atoms. Said divalent salt is advantageously a calcium salt such as $CaCO_3$, $Ca(HCO_3)_2$, $CaCl_2$, $Ca(NO_3)_2$, $CaSO_4$, $Ca_3(PO_4)_2$ $CaBr_2$, $CaI_2$, $Ca(OH)_2$, $Ca(NO_2)_2$, $CaC_2O_4$, $Ca(H_2PO_4)_2$ and hydrates thereof, preferably $CaCl_2$. Said second salt may be NaCl, NaBr, NaI, $Na_2SO_3$, $Na_2SO_4$, $NaHCO_3$, $Na_2CO_3$, preferably NaCl. Lithium salts, e.g. LiBr, could also be used if desired. The concentration of said divalent salt is preferably between 1 and 50 mM, still preferably between 10 and 30 mM, even preferably 20 mM. The concentration of said second salt is preferably between 1 and 200 mM, still preferably between 20 and 150 mM, still preferably 50 mM.

In a preferred embodiment, the aqueous solution comprises $CaCl_2$ and NaCl. The concentration of $CaCl_2$ may be e.g. between 1 and 50 mM, preferably between 10 and 30 mM, preferably 20 mM. The concentration of NaCl may be e.g. between 1 and 200 mM, preferably between 20 and 150 mM, still preferably 50 mM.

In a particular embodiment, the concentration of fibrinogen in the aqueous solution may be between 2 and 20 mg/mL, preferably between 3 and 12 mg/mL, more preferably 10 mg/mL, and the concentration of silk fibroin may be between 2 and 100 mg/mL, preferably between 5 and 60 mg/mL, more preferably 20 mg/mL.

In a preferred embodiment, the silk fibroin/fibrin ratio in the mixture is 2/1 (w/w).

The peroxidase may be used in solution. Preferably, the peroxidase is the horseradish peroxidase.

In a particular embodiment, the concentration of the peroxidase in the aqueous solution may be between 10 and 1000 U/mL, preferably 100 U/mL, with 1 U corresponding to the amount of enzyme which oxidizes 1 $\mu$mol of 2,2'-azino-bis(3-ethylbenzothiazoline-6-sulphonic acid) (ABTS) at pH 6.0 and 25°C.

The oxidizing agent in contact with the peroxidase triggers the gelation of silk fibroin. Thrombin is an enzyme which triggers the gelation of fibrin by its action on fibrinogen.

The oxidizing agent, may be oxygen, hydrogen peroxide, an organic peroxide, a metal peroxide, a hydroperoxide, a peracid, or a metallic oxidant. Preferably, the oxidizing agent is hydrogen peroxide. The oxidizing agent may be used in solution, or may be used pure, in a solid, liquid, gaseous state.

In a particular embodiment, the peroxidase is the horseradish peroxidase and the oxidizing agent is hydrogen peroxide.

The concentration of the oxidizing agent in the aqueous solution may be e.g. between 0.001 and 0.5%, preferably 0.01 % (w/v).

In a preferred embodiment, the peroxidase is the horseradish peroxidase and has a concentration in the aqueous solution of 100 U/mL (assayed with ABTS) and the oxidizing agent is the hydrogen peroxide and has a concentration in the aqueous solution of 0.01 % (w/v).

The concentration of thrombin in the aqueous solution may be e.g. between 0.04 and 4 U/mL, preferably 0.4 U/mL. Units correspond to NIH units. One unit is determined by comparison with a standard curve prepared using the Bureau of Biologics standard.

The mixture may be obtained by a successive or a simultaneous addition of any or all the compounds involved. Preferably, the compounds are added successively in the aqueous solution, in the following order: fibrinogen, the silk fibroin solution, the peroxidase, the oxidizing agent and then thrombin. Alternatively, the silk fibroin solution the peroxidase and the oxidizing agent are contacted, shortly before adding fibrinogen and thrombin.

The incubation temperature may be set between 2 and 50 °C, preferably between 30 and 40 °C, even more preferably at 37 °C. In a particular embodiment, the temperature of the aqueous solution, the fibrinogen, the silk fibroin solution, the peroxidase, the oxidizing agent and thrombin is equilibrated at 37 °C prior to mix.

The incubation may be carried out for 30 minutes to 24 hours, preferably 2 hours to 6 hours, more preferably 4 hours.

In a preferred embodiment, the method comprises the steps of:

(i) preparing a mixture comprising

- fibrinogen,
- a silk fibroin solution,
- and a peroxidase,

in an aqueous solution;

(ii) adding an oxidizing agent and thrombin (simultaneously or in any order), and incubating under conditions allowing the IPN of fibrin and silk fibroin to form.

The oxidizing agent and thrombin may be added successively in any order or simultaneously. In a preferred embodiment, the oxidizing agent is added before thrombin.

In this embodiment, the temperature in step (i) and the incubation temperature in step (ii) may be independently set between 2 and 50 °C, preferably between 30 and 40 °C, even more preferably at 37 °C. Preferably, the temperature in step (i) and the incubation temperature in step (ii) are the same.

The incubation in step (ii) may be carried out for 30 minutes to 24 hours, preferably 2 hours to 6 hours, more preferably 4 hours.

After incubation, the obtained gel material is preferably rinsed. An example of solution employed for the rinsing step is PBS or HEPES 10 mM in water, pH 7.4, NaCl 50 mM, $CaCl_2$ 20 mM.

Said "peroxidase" method may be carried out in any container, such as a beaker, a crystallizer, a flask, a test tube, a mold. Said mold may consist of standard 12 wells cell culture plate, with cleaned circular PTFE chips (16 mm diameter) placed in alveoli. The container is preferably closed.

The gel material obtainable by said "peroxidase" method is particularly advantageous as a support for cells. Indeed it is degradable by thermolysin and allows cells to adhere, spread, and proliferate.

Said gel material may be a material for tissue engineering, such as cartilage, muscle, skin, tendon/ligament, bone, or cornea, preferably for skin tissue engineering. In a particular embodiment, said gel material is a skin substitute in the treatment of burns and skin repair. In another embodiment, said gel is a skin model for research and screening applications including penetration studies, pigmentation studies and toxicity, corrosivity and irritation testing, particularly in cosmetic.

Said gel material may further comprise a drug. It is then useful for drug delivery, preferably sustained release. In a preferred but non-limiting embodiment, said drug is hydrosoluble and is advantageously added during rinsing. Alternatively it may be added at any step during the manufacturing of the gel.

The "alcohol" method:

An alternative method for obtaining a gel material comprising an interpenetrating polymer network (IPN) of fibrin and silk fibroin comprises the steps of:

(i) preparing a mixture comprising fibrinogen and a silk fibroin solution in an aqueous solution;
(ii) adding thrombin to the mixture prepared in (i) and incubating under conditions allowing fibrinogen to convert into fibrin fibers that at least partially crosslink;
(iii) contacting the reaction mixture obtained in (ii) with an alcohol and incubating under conditions allowing the IPN to form.

A particular embodiment of this method is shown on Figure 19.

The aqueous solution may comprise at least one salt, and preferably the aqueous solution comprises one salt and optionally further comprises a second salt.

More preferably, the aqueous solution comprises a divalent salt and optionally further comprises a second salt. Said divalent salt is advantageously a calcium salt such as $CaCO_3$, $Ca(HCO_3)_2$, $CaCl_2$, $Ca(NO_3)_2$, $CaSO_4$, $Ca_3(PO_4)_2$ $CaBr_2$, $CaI_2$, $Ca(OH)_2$, $Ca(NO_2)_2$, $CaC_2O_4$, $Ca(H_2PO_4)_2$ and hydrates thereof, preferably $CaCl_2$. Said second salt may be NaCl, NaBr, NaI, $Na_2SO_3$, $Na_2SO_4$, $NaHCO_3$, $Na_2CO_3$, preferably NaCl. Lithum salts, such as LiBr, may also be used if desired. The concentration of said divalent salt is between 1 and 50 mM, preferably between 10 and 30 mM, preferably 20 mM. The concentration of said second salt may be between 1 and 200 mM, preferably between 20 and 150 mM, preferably 50 mM.

In a preferred embodiment, the aqueous solution comprises $CaCl_2$ and NaCl. The concentration of $CaCl_2$ may be between 1 and 50 mM, preferably between 10 and 30 mM, preferably 20 mM. The concentration of NaCl may be between 1 and 200 mM, preferably between 20 and 150 mM, preferably 50 mM.

The concentration of fibrinogen in the aqueous solution may be between 2 and 20 mg/mL, preferably between 3 and 12 mg/mL, more preferably 10 mg/mL, and the concentration of silk fibroin may be between 2 and 100 mg/mL, preferably between 5 and 60 mg/mL, more preferably 20 mg/mL.

In a preferred embodiment, the silk fibroin/fibrin ratio in the mixture is 2/1 (w/w).

Step (ii) consists of adding thrombin to the mixture prepared in (i) and incubating under conditions allowing fibrinogen to convert into fibrin fibers that at least partially crosslink.

During step (ii), thrombin triggers the gelation of fibrin by its action on fibrinogen. The concentration of thrombin in the aqueous solution in step (ii) may be e.g. between 0.04 and 4 U/mL, preferably 0.4 U/mL. Units correspond to NIH units. One unit is determined by comparison with a standard curve prepared using the Bureau of Biologics standard.

Steps (i) and (ii) may occur successively. In this embodiment, fibrinogen, the silk fibroin solution, and thrombin are added successively in this given order.

The incubation temperature in step (ii) may be set between 2 and 50 °C, preferably between 30 and 40 °C, even more preferably at 37 °C. In a particular embodiment, the incubation temperature is the same as in step (i).

The incubation in step (ii) may be carried out for 30 minutes to 24 hours, preferably 2 hours to 6 hours, more preferably 4 hours.

A material comprising a semi-interpenetrating polymer network (semi-IPN) is obtained, since only fibrin gelation has occurred. Semi-IPN is distinct from IPN, since only one of the two networks forms.

According to the method of the present invention, the gelation of the second constituent, namely silk fibroin, is triggered by the presence of an alcohol.

In step (iii), the material obtained in step (ii) is contacted with an alcohol. Said material may be immersed in a volume of an alcohol solution, said alcohol solution being a solvent comprising an alcohol. The solvent may be, for instance, water or an alcohol.

In a particular embodiment, the solvent of the alcohol solution is water, and the concentration of alcohol in the solvent is between 5 and 100 % (v/v), preferably between 20 and 60%, more preferably 40 %.

The volume of alcoholic solution may be dependent on the volume of the material obtained in step (ii). In a particular embodiment, the volume of alcohol solution is between 0.5 and 1000 times the volume of the material, preferably between 1 and 100 times, more preferably 10 times.

Alternatively, the alcohol solution may be added on the material.

The alcohol used in step (iii) may be selected from the group consisting of methanol, ethanol, isopropanol or any linear or branched (C3-C12-alkyl)-alcohol, and is preferably ethanol.

The incubation temperature in step (iii) may be set between 2 and 50 °C, preferably between 30 and 40 °C, even more preferably at 37 °C. In a particular embodiment, the incubation temperature is the same as in step (ii).

The incubation in step (iii) may be carried out for 30 minutes to 24 hours, preferably 2 hours to 6 hours, more preferably 3 hours.

After incubation, a gel material, which comprises an interpenetrated polymer network of fibrin and silk fibroin, is obtained. Said gel material is preferably rinsed. An example of solution employed for the rinsing step is PBS or HEPES 10 mM in water, pH 7.4, NaCl 50 mM, CaCl$_2$ 20 mM.

Said "alcohol" method may be carried out in any container, such as a beaker, a crystallizer, a flask, a test tube, a mold. Said mold may consist of standard 12 wells cell culture plate, with cleaned circular PTFE chips (16 mm diameter) placed in alveoli. The container is preferably closed.

The gel material obtainable by said "alcohol" method is particularly useful as a filling material.

Said gel material may further comprise a drug. It is then useful for drug delivery, preferably sustained release.

In a first embodiment, said drug is insoluble or poorly soluble in water, but soluble in alcohol. A drug is insoluble in water if it has a solubility in water typically less than 0.5 g/L at 25 °C.

A drug is poorly soluble in water if it has a solubility in water typically between 0.5 and 1 g/L, at 25 °C.

A drug is soluble in alcohol if it has a solubility in alcohol typically higher than 1 g/L, preferably higher than 5 g/L, at 25 °C.

Said drug is advantageously incorporated during step (iii). Said drug may be added to the alcohol solution, prior to contacting with the material obtained in step (ii). After said contact, a suitable incubation time may be applied so that said drug diffuses into the whole material volume.

Said drug may be incorporated during step (i) or (ii) and the material obtained in step (ii) may be immersed in the alcohol solution containing said drug.

In another embodiment, said drug is hydrosoluble and is advantageously added after step (iii), typically during the rinsing step.

The invention will also be described in further detail in the following examples, which are non-limiting.

Examples

Abbreviations

2/1 SF/Fb IPN refers to a gel material comprising an interpenetrating polymer network (IPN) of silk fibroin (SF) and fibrin (Fb), the ratio silk fibroin/fibrin being 2/1.

1% Fb SN refers to a gel material comprising a simple network (SN) of fibrin (Fb), the concentration of fibrin being 1% (w/v)

2% SF SN refers to a gel material comprising a simple network (SN) of silk fibroin (SF), the concentration of silk fibroin being 2% (w/v).

1% Fb Semi-IPN refers to a gel material comprising a semi-interpenetrating polymer network of fibrin (Fb), the concentration of fibrin being 1% (w/v). Said 1% Fb semi-IPN differs from 1% Fb SN in that it comprises Silk Fibroin which is not in a gelified state. 1% Fb SN does not comprise Silk Fibroin.

HEPES is 4-(2-hydroxyethyl)-1-piperazineethanesulfonic acid

PTFE is polytetrafluoroethylene

BCA means Bicinchoninic Acid

PBS is Phosphate Buffered Saline

TBS is Tris-buffered saline

AP is alkaline phosphatase

BSA is bovine serum albumin

DAPI is 4',6-diamidino-2-phénylindole

SDS is Sodium Dodecyl Sulfate

SDS-PAGE is Sodium Dodecyl Sulfate-PolyAcrylamide Gel Electrophoresis

B-MSH is β-mercaptoethanol

**1. Characterization methods**

1.1. Protein soluble extractable fraction

To determine protein extractable fraction, 200 μL materials are synthesized. Right after synthesis, they are immersed in 4 mL HEPES buffer (HEPES 10 mM pH 7.4) and incubated at 37 °C under mild agitation (150 rpm) for 72 hours. After what, proteins in the solution are dosed with BCA method. Some materials expulse solution at synthesis which may contain proteins. Then, this solution is recovered from the PTFE chip with a Kimwipe®, which is immersed in 1 mL HEPES buffer for at least 1 hour at room temperature. Finally, the solution is recovered and proteins are dosed with BCA method.

Protein concentration dosage by BCA method is performed with Sigma-Aldrich BCA kit, in 96 wells microplate, according to its protocol.

Silk Fibroin and Fibrinogen are used as standards. Standards with concentrations varying from 0 to 1 mg/mL are prepared in HEPES buffer.

Working reagent is used according to provider protocol (BCA1 product, Sigma-Aldrich). In each wells of a 96 wells plate, 25 $\mu$L of standard or sample is deposited, followed by 200 $\mu$L of working reagent. The microplate is incubated at 37 °C for 30 min. After what, the OD 570 nm of every well is measured with a microplate reader (ELx800 (BioTek) or Xenius (SAFAS)).

From these values, the total mass of extracted proteins is determined. Finally, protein soluble extractable fraction is defined as the ratio of extracted proteins mass over initially introduced protein mass. The used formula is

$$Protein\ soluble\ extractable\ fraction = \frac{m(extracted\ proteins)}{m(initially\ introduced\ proteins)}$$

### 1.2. Viscoelastic properties of materials

Storage and loss moduli (named G' and G" respectively) are measured using a MC301 rheometer (Anton Paar) with a CTD 450 furnace and an anti-evaporation device.

a) In situ analysis

In situ gelation of materials is followed at 37 °C, using an aluminum smooth plane/plane 25 mm diameter geometry. The gap is set at 0.5 mm, and the materials are subjected to 1% strain at 1 Hz frequency for defined times.

The gelation point is usually considered reached when the G' becomes higher than the G". However, on all tested materials, this gelation point was never observed as G' was higher than G" even at the very beginning of measurement. Then, the gelation point was determined using the double tangent method on the G" curve. Another characteristic time was determined: the time when G' becomes higher than ten times the G", which corresponds to the obtaining of a cohesive gel.

b) Ex situ analysis

Ex situ measurements are performed at 37 °C using an aluminum smooth plane/plane 25 mm diameter geometry and on 700 $\mu$L materials, rinsed and swollen in synthesis buffer (HEPES, 50 mM NaCl, 20 mM CaCl$_2$). These materials have an about 25 mm diameter. The descent of the upper geometry is stopped as soon as 0.4 N normal force is detected, without post-control. Materials are then subjected to 0.1 % strain at 1 Hz frequency for 5 minutes. These parameters are in the linear domain of all tested materials.

### 1.3. Western-Blot

150 $\mu$L materials are synthesized, and rinsed/swollen in synthesis buffer (HEPES, 50 mM NaCl, 20 mM CaCl$_2$). The fibrin network is extracted from Fb SN or IPNs by incubating these materials in chemical degradation buffer (160 mM Tris pH 6.8, 8 M Urea, 2% (w/v) SDS, 2% (v/v) $\beta$-MSH) for 72 hours at 37 °C, under mild agitation (150 rpm).

Then, protein samples are subjected to migration on a 7% SDS-PAGE. After migration, proteins are transferred on a nitrocellulose membrane (BioRad) overnight at 8°C, under a constant 120 mV voltage in transfer buffer (25 mM Tris pH 8.8, 20% (v/v) Ethanol, 192 mM Glycine). The proper transfer was verified with red ponceau solution.

Then, the membrane is saturated with milk saturation buffer (3% powder milk (w/v) in PBS) at room temperature for 1 hour. After saturation, the membrane is incubated in a milk rinsing buffer (PBS, 1% (w/v) powder milk, 0.5% (v/v) Tween 20) containing the 500x diluted antihuman fibrinogen antibody at room temperature for 1 hour. Then, the membrane is rinsed 3 times for 10 min (room temperature) with milk rinsing buffer, prior being incubated for 1 hour at room temperature in milk rinsing buffer containing the 5000x diluted secondary antibody conjugated with AP (Anti rabbit IgG-AP). After this incubation, the membrane is once again 3 times rinsed with milk rinsing buffer for 10 min (room temperature), and then rinsed in TBS buffer (50 mM Tris pH 7.4, 150 mM NaCl).

Bands are revealed using the AP color Kit (BioRad), and the membrane is scanned with a GS-800 calibrated densitometer (BioRad), and image analyszed with QuantityOne software (BioRad).

1.4. Degradation of materials

300 $\mu$L materials are synthesized, and rinsed/swollen in synthesis buffer. Then, they are immersed in 3 mL of synthesis buffer containing 0.1 U/mL thermolysin (1 U/mL$_{mat}$) or 50 U/mL trypsin (500 U/mL$_{mat}$), and incubated at 37 °C under mild agitation.

At defined times, 10 $\mu$L of each sample are withdrawn and deposited in a 96 wells plate. At the end of the experiment, a maximum of 200 $\mu$L are withdrawn. Hence, this total does not exceed 10% of the initial solution volume. Then, 15 $\mu$L HEPES buffer is added to the 10 $\mu$L sample in each well. Between each point, the plate was frozen to stop the enzymatic reaction. Finally, at the end of the experiment, protein concentration of all samples is dosed with BCA method as previously described, using fibrinogen and silk fibroin as standards.

The degradation percentage at defined time is determined by the ratio of the mass of protein fragments released in the solution, over the initial mass of proteins.

1.5. Microscopic morphology

a) Confocal Laser Scanning Microscopy (CLSM)

To observe the microscopic morphology of the fibrin network in CLSM, 100 $\mu$L materials are synthesized and swollen in synthesis buffer. After what they are incubated in BSA saturation buffer (PBS, 1% (w/v) BSA for 1 hour at room temperature and rinsed 3 times for 10 min in rinsing buffer. Then, they are incubated in BSA rinsing buffer (PBS, 0.5% (w/v) BSA, 0.5% (v/v) Tween 20) containing anti-human fibrinogen FITC (Fluorescein IsoThioCyanate) conjugated antibody (diluted 100x) at room temperature for 3 hours. Finally, materials are rinsed 3 times at room temperature for 10 min in BSA rinsing buffer. They are conserved in PBS at 4 °C until analysis. All incubation and rinsing steps are performed protected from light. Materials are analyzed using a LSM 710 (Zeiss) CLSM following Nyquist rule. The oil-immersed x 40 and x 63 objectives are used (respective numeric aperture 1.2 and 1.4). The fibrin network is imaged using the Argon laser ($\lambda$ex 488 nm). Data are collected and treated with ImageJ software.

b) Scanning Electron Microscopy (SEM)

Microscopic morphology of materials was imaged using a FEG Gemini SEM 300 (Zeiss), with an acceleration tension of 5 kV, in partial void (VPSE) mode at 100 Pa, with collection of secondary electrons.

150 $\mu$L materials are synthesized and rinsed/swollen in pure water and do not require further preparation. For imaging, a material is placed onto a Peltier platinum at -25°C, and freezes very quickly. Then, the Peltier platinum is placed in the device void chamber. In the partial void at -25°C, the material starts to freeze-dry. Images are then acquired after about 5-10 minutes, when the water vapor is evacuated and does not hinder imaging.

1.6. Cell culture study

a) Cell culture, maintenance and conservation

In this study, FB-BJ cells are used, which are a lineage of dermis fibroblasts from newborn foreskin.

They are cultured using complete cell culture medium consisting in DMEM High glucose with Glutamax® (Gibco) supplemented with 20 U/mL penicillin and streptomycin, and 10% (v/v) fetal calf serum (FCS), in an incubator at 37 °C under wet atmosphere with 5% CO2. The medium is changed twice a week.

Cells are used at confluence and are detached from their support with trypsin-EDTA treatment at 37 °C (in the incubator) for few minutes (until all cells are detached). Then, the cell suspension is centrifuged at 200 g for 5 min, and the cell pellet is resuspended at desired concentration in complete cell culture medium.

The ATCC recommends to not use FB-BJ at passage higher than 15. Then, these cells were used with passage varying from 4 to 11.

To conserve cells on the long term, confluent cells are detached with trypsin-EDTA treatment at 37 °C (in the incubator), centrifuged at 200 g for 5 min, and the cell pellet is resuspended at 1 to 2.106 cells/mL in specific medium (DMEM high glucose + Glutamax®, with 20 U/mL penicillin and streptomycin, 50% (v/v) FCS and 10% (v/v) DMSO). The cell suspension is then pipetted into cryotubes (Corning), which are placed overnight at -80°C in a "Mr Frosty" (Nalgene) container, prior conservation in liquid nitrogen.

The thawing of cells is performed by immersing a cryotube in a bath at 37 °C, then cells are quickly put in culture in complete cell culture medium, which is changed after few hours.

b) Cell seeding on material

200 μL of 2/1 SF/Fb IPNs are synthesized in sterile conditions. However, materials are not unmolded from the PTFE chip right after the synthesis, but are placed in 12 well cell culture plate, rinsed and swollen in DMEM high glucose + Glutamax® with 20 U/mL penicillin and streptomycin, and then equilibrated in complete cell culture medium.
After at least 24 hours, the medium is withdrawn and 60 μL of a $4.10^5$ FB-BJ cells/mL solution (24 k cells/material) are carefully deposited on material surface. Then, the plate is placed in the incubator at 37 °C for 2 hours. After what, seeded materials are unmolded with a thin PTFE blade, and placed in 1 mL complete cell culture medium in 24 wells cell culture plates. FB-BJ cells are then cultured for 2 weeks.

c) Material contraction

200 μL materials are synthesized and seeded with FB-BJ cells as described in previous paragraph. Just after seeding, initial material diameter is measured by placing a graph paper behind the 24 well culture plate. Then, at defined times, the diameter is measured with the same method, and diameter values are converted into surface values. Finally, surface percentages at defined times are expressed as the ratio of the surface at this time over the initial surface value.

d) Cell viability

At defined time, cell culture medium in which seeded materials are incubated is withdrawn. Materials are rinsed with 1 mL PBS, and incubated in PBS containing calcein AM and ethidium homodimer I (obtained by mixing 1 mL PBS with 1 μL calcein AM and 0.5 μL ethidium homodimer I commercial solutions). Then, materials are placed in the incubator for 45 min, prior being rinsed with PBS. Finally, the materials are analyzed in CLSM. The 10 x objective is used, and both fluorophores are simultaneously detected at 488 nm for the calcein AM and 561 nm for the ethidium homodimer I. Attention is paid to protect samples from light before analysis.

**2. Reagent preparation**

2.1. Fibrinogen and thrombin

Under microbiological safety place (MSP), fibrinogen 1 g vial (Calbiochem) is suspended in 19 mL sterile HEPES 10 mM pH 7.4 (final concentration 5% w/v) at 37 °C without agitation until complete solubilization (about 3-5 hours). Then, solution is fractionated under MSP and stored at -20 °C. When needed, aliquots are thawed and equilibrated at 37 °C.
Under MSP, thrombin 1000 U vial (Calbiochem) is suspended in 1 mL sterile Tris 50 mM pH 7.4, 1 mg/mL BSA. Dissolution is fast, and after, the solution is then quickly diluted at 20 U/mL in same buffer, fractionated and stored at -20 °C. When needed, aliquots are thawed extemporaneously at room temperature just before its addition.

2.2 Silk Fibroin

The commercial Silk Fibroin (SF) solution at 40-60 mg/mL (Advanced Biomatrix) is kept at -80 °C until use. For aliquots preparation, the 20 mL vial is slowly thawed at 8 °C. After what the solution is fractionated under MSP, and aliquots are stored at -20 °C.
When needed, aliquots are thawed at room temperature. Then, aliquots are centrifuged at 7500 rpm for 1 minute at room temperature, to pellet eventual aggregates. The supernatant is recovered, and its pH is equilibrated to 7.5 ± 0.1 with HCl 0.1 M. Depending on the batch, the pH of the SF commercial solution may vary between 8 and 10, then, this acidification is mandatory, to ensure reproducibility. Then, the acidified SF solution is centrifuged at 7500 rpm for 1 minute at room temperature. Finally, the supernatant is recovered, and equilibrated at 37 °C.
Remaining SF solution is stored at 4 °C and could be used for the next 3 days.

2.3. Horseradish peroxidase and hydrogen peroxide

Horseradish peroxidase (HRP) (Sigma-Aldrich) aliquots (few mg) are prepared and stored at 4 °C. When needed, HRP is solubilized extemporarily at 1000 U/mL in pure water at room temperature, and kept at room temperature until use.
Hydrogen peroxide, a pharmaceutical product (Eau Oxygénée 10 volumes, Laboratoires Gilbert) is kept at 4 °C. For each experiment, about 1 mL is quickly withdrawn, diluted ten times, and kept at room temperature until use (protected from light).

## 3. Preparation of the gel materials

### 3.1. Presentation of the mold

The synthesis mold consists of standard 12 wells cell culture plate, with cleaned circular PTFE chips (16 mm diameter) placed in alveoli. The mold is kept in a hermetic wet chamber. Prior synthesis, the entire mold temperature is equilibrated at 37 °C for at least 30 minutes.

Then, material precursor solutions are prepared, and desired volume is poured with a pipette onto PTFE chips. Then, the plate is covered with its lid, and the hermetic box tightly closed and placed in an oven at 37 °C for gelation.

After synthesis, PTFE chips onto materials are slightly adherent are recovered with thin tweezers, and materials are removed using a thin PTFE blade.

### 3.2 Preparation of a gel material though HRP-$H_2O_2$/thrombin method

The preparation of a gel material comprising an interpenetrating polymer network of fibrin and silk fibroin through HRP-$H_2O_2$/thrombin is performed as follows. All reagents are thawed/prepared as mentioned in previous section, and their temperature is equilibrated at 37 °C for at least 30 minutes, except for horseradish peroxidase (HRP) and $H_2O_2$. Thrombin is thawed at the last moment. Then, material precursor solution is prepared by mixing components in that order: pure water, salts, fibrinogen, silk fibroin, HRP, $H_2O_2$, and thrombin.

Table 1: Protocol for the preparation of 1 mL of 1 % Fb SN, 2% SF SN and 2/1 SF/Fb IPN, through HRP-$H_2O_2$/ thrombin method

| Compone nt | Initial concentratio n | 1 % Fb SN | | 2% SF SN | | 2/1 SF/Fb IPN | |
|---|---|---|---|---|---|---|---|
| | | Final concentratio n | Volum e ($\mu$L) | Final concentratio n | Volum e ($\mu$L) | Final concentratio n | Volum e ($\mu$L) |
| Pure water | - | QS 1000 $\mu$L | 730 | QS 1000 $\mu$L | 416.7 | QS 1000 $\mu$L | 196.7 |
| NaCl, $CaCl_2$ | 1 M, 0.4 M | 50, 20 mM | 50 | 50, 20 mM | 50 | 50, 20 mM | 50 |
| Fibrinogen | 5% | 1 % | 200 | - | - | 1 % | 200 |
| Silk Fibroin | 5% | - | - | 2% | 400 | 2% | 400 |
| HRP | 1000 U/mL | - | - | 100 U/mL | 100 | 100 U/mL | 100 |
| $H_2O_2$ | 0.3 % | - | - | 0.01 % | 33.3 | 0.01 % | 33.3 |
| Thrombin | 20 U/mL | 0.4 U/mL | 20 | - | - | 0.4 U/mL | 20 |

Then, desired volume of material precursor solution is pipetted onto the surface of PTFE chips, and the entire mold is placed in an oven at 37°C for 4 hours. After this step, materials are recovered, immersed in synthesis buffer, and conserved at 4°C.

### Gelation studies:

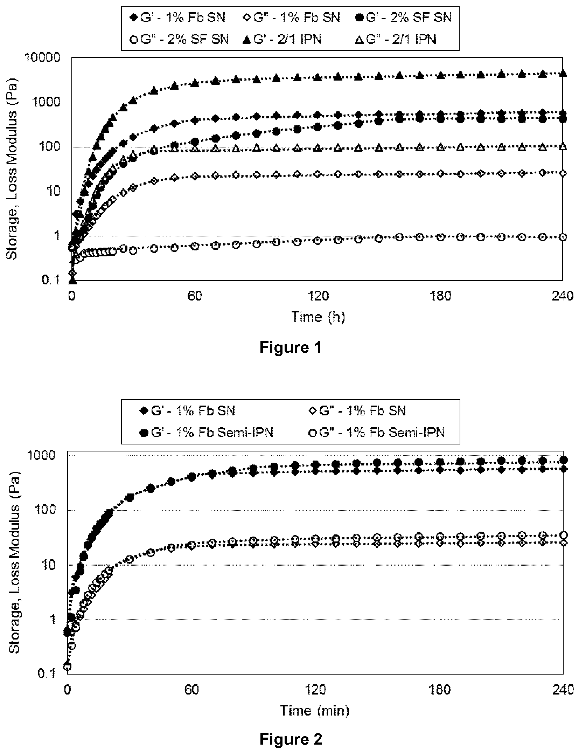

As shown in Figure 1, the gelation time for 2/1 SF/Fb IPN was about 5 minutes, and cohesive gel was obtained in about 11 minutes. The plateau was reached in about 1.5 hour, and at 4 hours, storage and loss moduli were about 4600 and 100 Pa.

For 1% Fb SN, the gelation time was about 3 minutes, and a cohesive gel was obtained in about 10 minutes. The plateau was reached in about 1 hour, and after 4 hours, storage and loss moduli were about 550 and 25 Pa respectively.

For 2% SF SN, the gelation time was about 5 minutes, and cohesive gel was obtained also in about 10 minutes. The plateau was reached in about 3 hours, with storage and loss moduli after 4 hours of about 400 and 1 Pa. The loss modulus value is very low, but it is not surprising, as the SF is a hydrophobic protein that then binds low water amount, what is likely to decrease the viscosity.

Therefore, the simultaneous gelation of both networks has no incidence on the phase transition occurrence, but has strong synergistic effect on the storage and loss moduli. This is a first relevant insight of the formation of an IPN architecture.

3.3. Preparation of a gel material though ethanol/thrombin method

The preparation of a gel material comprising an interpenetrating polymer network of fibrin and silk fibroin through ethanol/thrombin is performed as follows.

All reagents are thawed/prepared as mentioned in previous section, and their temperature is equilibrated at 37 °C for at least 30 minutes. Thrombin is thawed at the last moment. Then, a material precursor solution is prepared by mixing components in that order:

- for 2% SF SN: pure water, salts, ethanol and silk fibroin.
- for 1% Fb SN: pure water, salts, fibrinogen and thrombin.
- for 2/1 SF/Fb IPN: pure water, salts, fibrinogen, silk fibroin and thrombin.

Table 2: Protocol for the preparation of 1 mL of 1% Fb SN, 2% SF SN and 2/1 SF/Fb IPN through ethanol/thrombin method

| Compone nt | Initial concentratio n | 1 % Fb SN | | 2% SF SN | | 2/1 SF/Fb IPN | |
|---|---|---|---|---|---|---|---|
| | | Final concentratio n | Volum e ($\mu$L) | Final concentratio n | Volum e ($\mu$L) | Final concentratio n | Volum e ($\mu$L) |
| Pure water | - | QS 1000 $\mu$L | 730 | QS 1000 $\mu$L | 150 | QS 1000 $\mu$L | 330 |
| NaCl, CaCl$_2$ | 1 M, 0.4 M | 50, 20 mM | 50 | 50, 20 mM | 50 | 50, 20 mM | 50 |
| Fibrinogen | 5% | 1 % | 200 | - | - | 1 % | 200 |
| Silk Fibroin | 5% | - | - | 2% | 400 | 2 % | 400 |
| Ethanol | 100 % | - | - | 40 % | 400 | - | - |
| Thrombin | 20 U/mL | 0.4 U/mL | 20 | - | - | 0.4 U/mL | 20 |

Then, desired volume of material precursor solution is poured with a pipette onto the surface of PTFE chips, and the entire mold is placed in an oven at 37 °C for 4 hours.

After this step, 2/1 SF/Fb IPN are recovered, and immersed in 10 times their volume of 40% (v/v) ethanol, for 3 hours at 37 °C.

Finally, materials are extensively rinsed in synthesis buffer, and conserved at 4 °C.

Gelation studies:

The SF/Fb IPN through ethanol/thrombin is obtained with a sequential synthesis. Indeed, even at low concentration (<10%), ethanol induces fibrinogen precipitation. Therefore, the 1% Fb network is first synthesized, in presence of 2% SF (semi-IPN), and it is immersed later in ethanol to induce SF gelation.

First, it was investigated whether the presence of 2% SF have an influence on the gelation of 1% Fb. Gelation kinetics of 1% Fb SN and 1% Fb Semi-IPN (containing 2% SF in its liquid phase) were studied by rheology (see Figure 2).

Without 2% SF, 1% Fb SN turned to a gel in about 3 minutes, and in a cohesive gel in about 10 minutes. The plateau was reached in about 1 hour, and after 4 hours, storage and loss moduli were about 550 and 25 Pa respectively.

In presence of 2% SF in the liquid phase (1% Fb Semi-IPN), the gelation was increased to about 5 minutes. The time to obtain a cohesive gel was also extended to about 15 minutes. In addition, the plateau was reached in 2 hours, and after 4 hours, storage and loss moduli are about 800 and 35 Pa (respectively).

Therefore, the presence of SF slightly delays Fb gelation, but at 4 hours, storage and loss moduli are similar. It appeared that the presence of SF has minor incidence on Fb gelation kinetics.

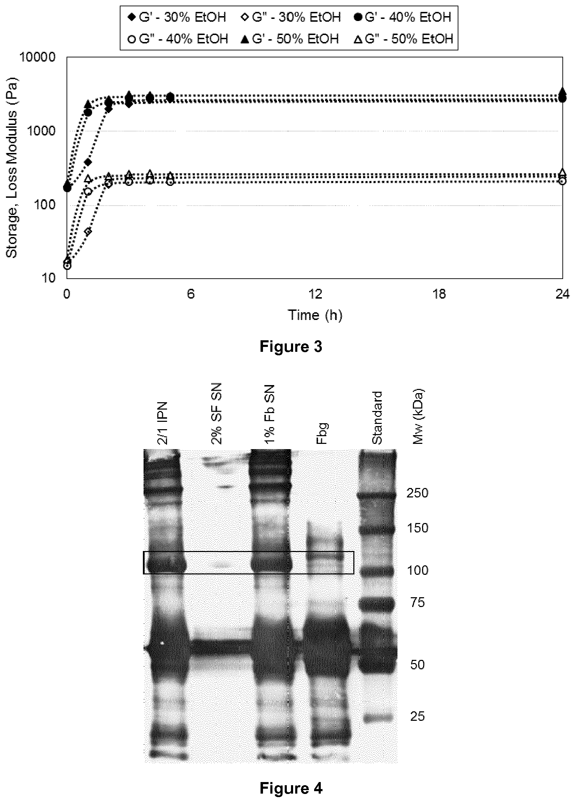

The semi-IPN was then immersed in 10 times their volume of ethanol with 3 different concentrations (30, 40, 50% v/v), and at defined times, storage and loss moduli were measured with the rheometer (see Figure 3).

With 30% ethanol, SF gelation starts slowly, but reaches a maximum in 4 hours, with storage and loss moduli of about 2700 and 250 Pa. After this time, viscoelastic properties hardly increase, even up to 24 hours.

With 40 and 50% ethanol, the gelation is faster, reaching a maximum in 3 hours with storage and loss moduli of about 2800 and 200 Pa (respectively) for 40% ethanol, and of 3100 and 250 Pa with 50% ethanol. After this time, viscoelastic properties do not increase anymore.

Thus, with all ethanol concentrations similar storage moduli ($\sim$ 3000 Pa) and loss moduli (-250 Pa) are obtained. However, the highest the ethanol concentration is, the fastest is the gelation, even if kinetics are comparable with 40 and 50%.

**4. Characterization of the gel material obtained through ethanol/thrombin method**

4.1. IPN architecture

The 2/1 SF/Fb IPN is very resistant and handable, consistently with the measured rheological values.

To investigate the formation of the Fb network, 1% Fb and 2% SF SNs and 2/1 SF/Fb IPN were immersed in a chemical degradation solution for 72h at 37°C. After what, supernatants were analyzed in Western Blot using polyclonal anti-fibrinogen antibody. A solution of 1% fibrinogen was used as a control (see Figure 4).

On the membrane, the presence of the γ-γ band at about 105 kDa is observed on 1% Fb SN and 2/1 SF/Fb IPN lanes, and is absent in fibrinogen and 2% SF SN. Therefore, the presence of Fb network within the IPN is then unquestionable.

After the incubation, the 1% Fb SN was completely dissolved, confirming its physical nature. On the other hand, the 2% SF SN and the 2/1 SF/Fb IPN were not solubilized and the remaining material presented the same volume. This suggests that these two materials contain a gel formed through bonds which are not reversible through urea, SDS and β-MSH treatment. This is surprising, as ethanol induces the gelation through formation of intermolecular β-sheets, and then, a physical gelation.

Nevertheless, as the 2% SF SN and the 2/1 SF/Fb IPN was not solubilized after the incubation in presented the same volume, it indirectly confirms the presence of the SF network within the IPN.

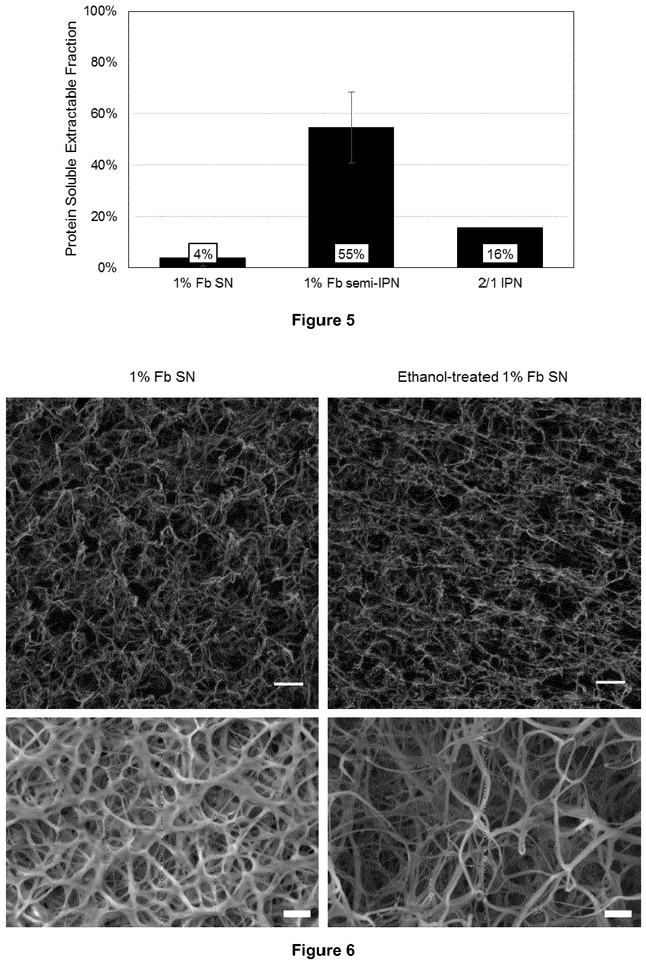

This observation on formation of the SF network is consistent with the fact that when the 1% Fb semi-IPN is immersed in 40% ethanol, its storage modulus increase sharply. In addition to these qualitative aspects, quantitative aspects were assessed by investigating protein soluble extractable fraction. Briefly, 1% Fb, 1% Fb semi-IPN, and 2/1 SF/Fb IPN were synthesized, and immersed in HEPES immediately after synthesis, and incubated at 37°C under mild agitation for 3 days. After incubation, extracted protein concentration was dosed with BCA method (see Figure 5).

Protein extractable fractions from the 1% Fb SN of about 4% were observed.

On the other hand, the protein soluble extractable fraction from the 1% Fb semi-IPN are about 55% and those of the 2/1 SF/Fb IPN 16%. Considering that the presence and gelation of SF does not have dramatic influence on Fb gelation (see Figure 2), the reduction of proteins extracted after immersion of the semi-IPN in ethanol from 55 to 16% indicates formation of the SF network in the material. However, SF soluble extractable fraction in the IPN are rather high.

Therefore, the presence of the SF network within the IPN is demonstrated.

4.2. Microscopic morphology

a) Influence of ethanol on Fb network

1% Fb SNs synthesized, and were immersed (or not) in 40% ethanol for 3 hours, to mimic the IPN synthesis conditions. After what they were extensively rinsed to remove ethanol, and imaged in SEM (Scanning Electron Microscopy), or immunostained with a polyclonal anti-fibrinogen antibody and imaged in CLSM (Confocal Laser Scanning Microscopy) (see Figure 6).

On both CLSM and SEM images, the characteristic fibrous structure of Fb can be observed, with homogeneous distribution in both conditions. Even if differences in fibers diameter and porosity can be observed, the overall structure is similar.

Therefore, the 40% ethanol treatment has no dramatic effects on the structure of the Fb network.

b) Confocal Laser Scanning Microscopy (CLSM) study of 2/1 SF/Fb IPN

1% Fb SN, 2% SF SN and 2/1 SF/Fb IPN were synthesized, rinsed and swollen in synthesis buffer, immunostained with a polyclonal anti-fibrinogen antibody and imaged in CLSM. On this images, in addition to the Fb network, the topography was also imaged, using the reflection mode.

For 1% Fb SN, the topography signal shows no other clear structure than the homogeneous and fibrous Fb network. For the 2% SF SN, no Fb network is detected, but the topography signal shows a material with no distinct, yet heterogeneous structure.

For the 2/1 SF/Fb IPN, two different structures are observed. The first is the Fb network, which presents its characteristic fibrous structure, homogeneously distributed. It appears denser than that of the 1% Fb SN which is consistent with the presence 2% SF which densifies the IPN protein content. The topography signal reveals another structure, which is then assumed to be SF with no distinct shape and heterogeneously distributed within the Fb network. It should be mentioned that SF is a very low antigenic protein, so that there is no available anti-SF antibody, and it cannot be imaged in CLSM through immunostaining.

c) Scanning Electron Microscopy (SEM) study of 2/1 SF/Fb IPN

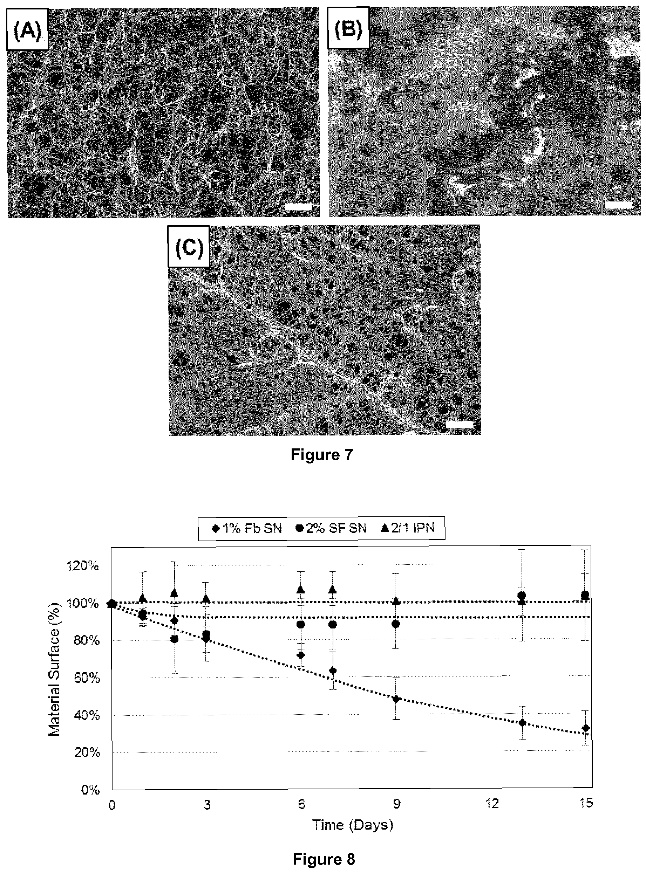

1% Fb SN, 2% SF SN and 2/1 SF/Fb IPN were synthesized, rinsed and swollen in pure water, and imaged with no further preparation (see Figure 7). The characteristic fibrous structure of the Fb network can be observed on the 1% Fb SN (A), with a homogeneous distribution. The overall morphology of the 2% SF SN (B) appears heterogeneous, with no distinct structures or shapes. Finally in the 2/1 SF/Fb IPN (C), the fibrous Fb network can also be observed. But it appears that its pores are partially and heterogeneously filled with another structure, assumed to be the SF network.
Therefore, the presence of the SF network within the IPN was also demonstrated, especially in SEM.

4.3. Cell culture study

a) Material contraction in the presence of fibroblasts

200 $\mu$L (10-12 mm diameter) of 1% Fb SN, 2% SF SN and 2/1 SF/Fb IPN were synthesized, rinsed and equilibrated in cell culture media (DMEM high glucose, Fetal Calf Serum 10%). After what $20.10^3$ Fb-BJ cells were seeded on their surface. At defined times, the diameter of materials were measured, and converted as surfaces and then to percentages to that of initial time (see Figure 8).
The 1% Fb SN strongly contracted in presence of fibroblasts, losing half of its surface in about 9 days, and 70% in 15 days. This contraction was expected, but in higher extent, as previous results in the lab showed comparable contraction in 2-3 days, yet with higher amount of cells. On the other hand, the 2% SF SN almost not contracted, losing a maximum of 10% of its surface, assumedly due to its high $\beta$-sheet density. And finally, the 2/1 SF/Fb IPN did not contract at all, preserving globally the entirety of its surface.
At 15 days, the presence of cells was confirmed through DAPI/Phalloïdin staining (staining of cell nucleus and actine cytoskeleton) on all materials.
In the presence of fibroblasts, the 1% Fb SN contracts strongly as expected, but the 2% SF SN and the 2/1 SF/Fb IPN do no contract. Thus, the presence of SF network in an IPN architecture with Fb network prevents its contractile behavior in presence of fibroblasts.

b) Viability

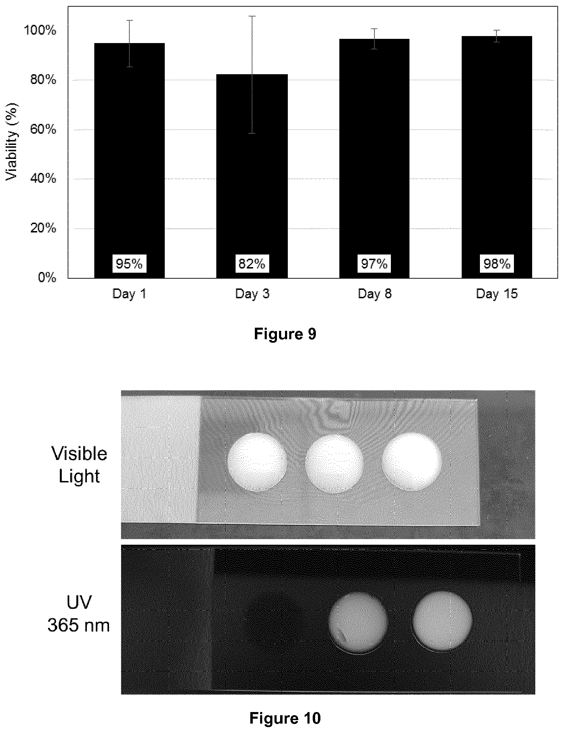

The cell viability was assessed at days 1, 3, 8 and 15 Live/Dead® method, and by seeding $20.10^3$ Fb-BJ on 200 $\mu$L 2/1 SF/Fb IPN (see Figure 9).
Despite a usual lower viability (~82%) at 3 days, Fb-BJ cells viability was at least 95% at days 1, 8 and 15. Therefore, the 2/1 SF/Fb IPN appears to be not cytotoxic for fibroblasts, as viability figures were higher at all times.

**5. Characterization of the gel material obtained through HRP-H$_2$O$_2$/thrombin method (the so-called "peroxidase method")**

5.1. IPN architecture

When manipulated, the 2/1 SF/Fb IPN is perfectly and easily handable, what is consistent with its viscoelastic properties.
HRP catalyzes the formation of a dityrosine between side chains of two tyrosines. However, dityrosine are fluorescent when excited with UV 365 nm, and then their presence can be observed macroscopically. 1% Fb SN, 2% SF SN and 2/1 SF/Fb IPN were excited with UV 365 nm.
The 3 materials are white opaque under visible light. However, when placed under UV 365 nm, 1% Fb SN emits no fluorescence while 2% SF SN and 2/1 SF/Fb IPN display strong and homogeneous fluorescence (see Figure 10).
This evidenced the presence of dityrosine bonds within the 2% SF SN and the 2/1 SF/Fb IPN. In addition, fluorescence was homogeneous, suggesting a homogeneous distribution of dityrosines within the material volume (at least at the micrometer level). Therefore, a SF network is probably present within the IPN.

It could be questioned whether the HRP also crosslinks Fb with Fb or SF. Then, a fibrinogen solution was incubated with HRP and $H_2O_2$, and even after 5 hours, no gelation was observed, as well as the solution did not present macroscopic fluorescence at 365 nm. In addition, a 1% Fb SN was synthesized (through action of thrombin) in presence of HRP and $H_2O_2$ and did not present macroscopic fluorescence at 365 nm either.

Very few Fb-Fb or Fb-SF crosslinks may be formed, but their presence was disregarded, in light of these observations. Thus, the HRP action was assumed to be specific to SF gelation.

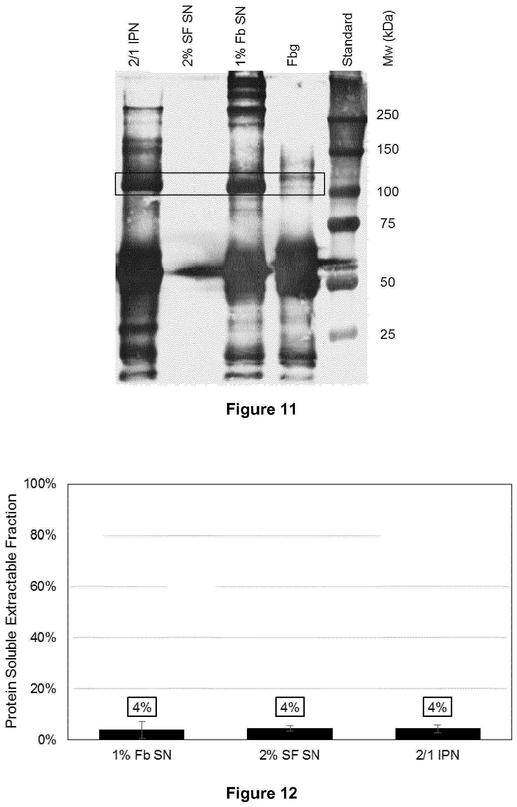

To investigate the formation of the Fb network, 1% Fb and 2% SF SNs and 2/1 SF/Fb IPN were immersed in a chemical degradation solution for 72h at 37°C. After denaturation, supernatants were analyzed in Western Blot using polyclonal anti-fibrinogen antibody. A solution of 1% fibrinogen was used as control (see Figure 11).

The γ-γ band (~ 105 kDa) was found in 1% Fb SN and 2/1 SF/Fb IPN lanes, while it was absent in fibrinogen and 2% SF SN lanes. Therefore, there is formation of a Fb network within the IPN.

It should be detailed that after the incubation, the 1% Fb SN was solubilized, confirming its physical gel nature, but neither the 2% SF SN nor the 2/1 SF/Fb were solubilized and globally kept their volume. As regards the 2% SF SN, it confirms its chemical gel nature. But as regards the 2/1 SF/Fb IPN, it confirms that it contains a chemical gel in addition to the Fb network. Therefore, the presence of the SF network within the IPN was also indirectly demonstrated.

Therefore, results demonstrated the presence of both Fb and SF networks within the IPN, but on a qualitative aspect. Quantitative aspects of the formation of these networks were investigated by measuring the protein extractibles from 1% Fb SN, 2% SF SN and 2/1 SF/Fb IPN (see Figure 12). It can be observed that for the three materials, protein extractibles are low, at about 4%. Therefore, within the IPN, the formation of a network does not hinder the formation of the other.

5.2. Microscopic morphology: CLSM & SEM

a) CLSM

1% Fb SN, 2% SF SN and 2/1 SF/Fb IPN were synthesized, the Fb network was immunostained with a polyclonal anti-fibrinogen antibody, and the overall topography was imaged through the reflection mode.

With the 1% Fb SN, the characteristic fibrous structure of Fb is observed, with a homogeneous repartition. In addition, the topography signal does not show other structures but the Fb network. On the other hand, as expected, no Fb network is observed in the 2% SF SN. However, the topography image shows a SF network with no distinct structures.

Finally, in the IPN, the Fb network is observed, with its characteristic fibrous structure. Therefore, the presence of SF does not alter the Fb network morphology. However, compared to that of the 1% Fb SN, it appears denser, but it is consistent with the presence of 2% SF, which densifies the structure.

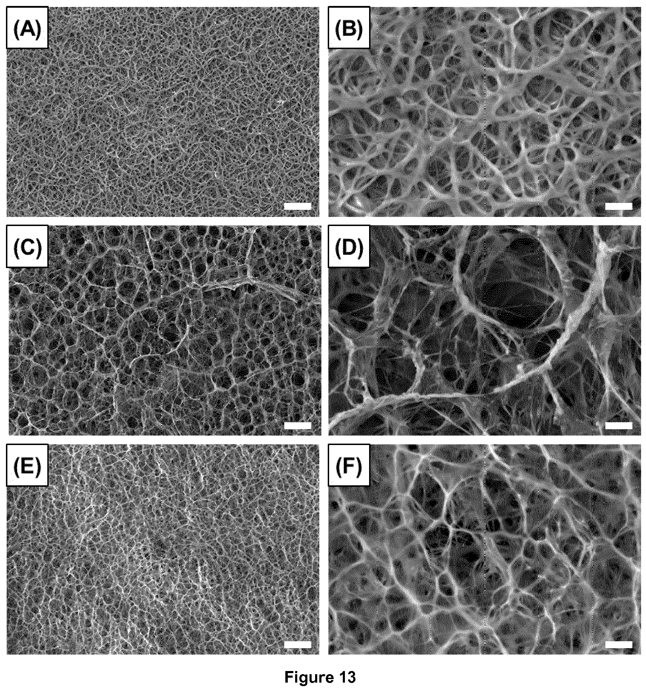

b) SEM (see Figure 13)

It can be observed that the 3 materials have a porous structure, with a relatively homogeneous repartition (A, C, D). Surprisingly, the 2% SF SN appears to have large pores (5-10 $\mu$m) with a globally penta- or hexagonal geometry, compared to the 1% Fb SN whose pores are smaller (2-5 $\mu$m) with no particular geometry. The structure of the 2/1 SF/Fb IPN presents also small pores (2-5 $\mu$m), but at the 10 $\mu$m scale, Fb and SF network cannot be differentiated.

At a larger magnification, it can be observed that the Fb network (B) presents interconnected fibers, resulting in a fibrous structure with 2-5 $\mu$m pores, but with no particular pattern. On the other hand, the details of the microscopic morphology of the 2% SF SN could be unraveled (D). It can be observed that its microstructure, with 5-10 $\mu$m pores is formed through the interconnection and clustering of nanofibers. Finally, even at a larger magnification, both SF and Fb networks could not be differentiated within the IPN (F). Indeed a fibrous structure is observed, but with pores size similar to that of the 1% Fb SF (2-5 $\mu$m), but with a geometry closer to that of the 2% SF SN.

Hence, it was observed that the 3 materials have a porous structure. However Fb and SF networks have different morphologies. More precisely, it was observed that the SF network presents 5-10 $\mu$m pores, with a relatively regular penta- or hexagonal geometry. In addition, its microstructure appeared to be obtained through clustering and interconnection of nanofibers. On the other hand, Fb network presents smaller pores (2-5 $\mu$m), with no particular geometry.

5.3. Degradation kinetics

Two proteases were used: thermolysin (ThL), which can hydrolyze both Fb and SF networks, and trypsin (Trp), which can selectively (based on *in silico* data) hydrolyze the Fb network.

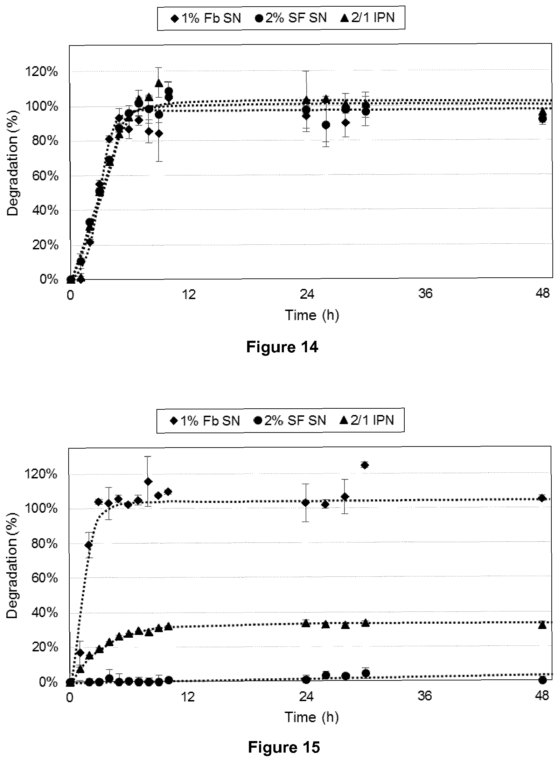

To investigate the degradation kinetics, 1% Fb SN, 2% SF SN and 2/1 SF/Fb IPN were incubated in ThL 1 U/mL$_{mat}$ or Trp 500 U/mL$_{mat}$ at 37°C. At defined times, the mass of released protein fragments is assessed with BCA assay, and reported to the initial protein mass in the material (see Figure 14).

All materials were quickly degraded by ThL in about 6 hours, with 50% degradation in 3 hours. No differences in the degradation behavior was observed, as they all were progressively solubilized. Yet, it is to detail that in the 2% SF SN and the 2/1 SF/Fb IPN, even after 96 hours, there were small particles remaining in the solution and resistant to the ThL action. HRP induces SF gelation through chemical crosslinks, and not by formation of intermolecular β-sheets. However, there were some insights of the presence of some of these. Therefore, it was assumed that these particles may be dense β-sheet-rich fragments of the SF network, which are then resistant ThL action.

Despite this observation, and as expected, the ThL can completely degrade both networks within the IPN.

Then, the experiment was also conducted with Trp 500 U/mL$_{mat}$ in same conditions (see Figure 15). With the Trp, results are very different. It can be observed that the 1% Fb SN was completely degraded (and solubilized) in 3 hours, with estimated half-life of 1.5 hours. On the contrary, the 2% SF SN was not degraded, even in 48 hours. Thus, and as expected, the Trp can hydrolyze the Fb network, but cannot degrade the SF network. As regards the 2/1 SF/Fb IPN, it can be observed that its degradation reaches a maximum of about 32% in 9 hours, what matches the proportion of Fb in the IPN. In addition, its half-life is estimated to about 3 hours, what is doubled compared to that of the 1% Fb SN (1.5 hours).

Therefore, the Trp can selectively hydrolyze the Fb network within the IPN. Moreover, the IPN architecture appears to delay its hydrolysis, at its half-life is almost doubled.

5.4 <u>Cell culture study</u>

a) Material contraction in the presence of fibroblasts

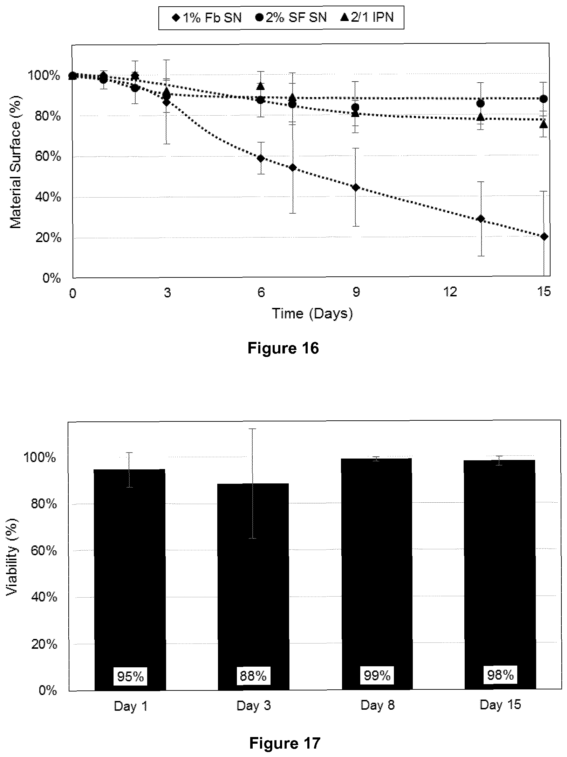

200 μL (10-12 mm diameter) 1% Fb SN, 2% SF SN and 2/1 SF/Fb IPN were synthesized, rinsed and equilibrated in cell culture media (DMEM high glucose, Fetal Calf Serum 10%). Then $20.10^3$ Fb-BJ cells were seeded on their surface. At defined times, the diameter of materials were measured, and converted to percentages to that of initial time (see Figure 16).

The 1% Fb SN contracted strongly in presence of fibroblasts, losing half of its surface in about 8 days, and 80% in 15 days. On the other hand, the 2% SF SN did not much contract, as it lost a maximum of 15% of its surface after 4 days. Finally, the 2/1 SF/Fb IPN slowly contracted, losing 20% of its surface in 9 days, and 25% in 15 days.

At 15 days, the presence of cells was confirmed through DAPI/Phalloïdin staining (staining of cell nucleus and actine cytoskeleton) on all materials.

Therefore, in presence of fibroblasts, the 1% Fb SN contracts strongly (50% of its surface is lost in a week) as expected, but the 2% SF SN and the 2/1 SF/Fb IPN contract less, losing a maximum of 25% in 15 days. Thus, the presence of SF network obtained through HRP action in an IPN architecture with Fb reduces its contractile behavior in presence of fibroblasts.

b) Fibroblasts culture on the surface of 2/1 SF/Fb IPN

$20.10^3$ Fb-BJ cells were seeded on the surface of 200 μL 2/1 SF/Fb IPN. At day 1, 3, 8 and 15, the cell viability, proliferation and morphology, as well as the evolution of the Fb network, and the production of mature ECM were assessed.

The cell viability was assessed at these times with Live/Dead® method (see Figure 17).

At day 1, 8 and 15, the viability was higher than 95%. The lowest viability was obtained as usual at day 3, with a value of 88%.

Therefore, the viability of Fb-BJ cells was particularly high when seeded on the surface of the 2/1 SF/Fb IPN.

Besides viability and proliferation, the cell morphology was also investigated at these times (nucleus stained with DAPI, actine cytoskeleton with Phalloïdin), as well as the evolution of Fb network (with Fb immunostaining).

At all times, cells presented a healthy morphology, even at day 1 and 3, with large nuclei and a fusiform actin cytoskeleton, suggesting that they have adhered and spread at surface of the material. In addition, the cell proliferation is clearly observable. As regards the Fb network, its presence is detected until day 8. At this time, it is still observed, but in the rare areas non-covered by the cells, and in small pieces among the cells. At day 15, only few remaining small pieces of the Fb network are detected among the cells.

Finally, the synthesis of fibronectin and type I collagen and fibronectin was also investigated through immunostaining at day 1, 3, 8 and 15.

It can be observed that the synthesis of fibronectin already started at day 1, and increased until day 15. On the other hand, the synthesis of type I collagen was detected since day 3, and its production increased until day 15.

Therefore, and considering that the Fb network was less and less detected, but the production of mature ECM proteins increased along time, these results suggest that cells remodeled the provisory Fb matrix into a mature ECM.

**Claims**

1.  A method for obtaining a gel material comprising an interpenetrating polymer network (IPN) of fibrin and silk fibroin, which method comprises contacting, in an aqueous solution, fibrinogen, a silk fibroin solution, a peroxidase, an oxidizing agent and thrombin and incubating under conditions allowing the IPN of fibrin and silk fibroin to form.

2.  The method according to claim 1, wherein the oxidizing agent is hydrogen peroxide.

3.  The method according to claim 1 or 2, wherein the peroxidase is the horseradish peroxidase.

4.  The method according to any of claims 1 to 3, wherein fibrinogen, the silk fibroin solution, the peroxidase, the oxidizing agent and thrombin are added successively in this given order.

5.  A method for obtaining a gel material comprising an interpenetrating polymer network (IPN) of fibrin and silk fibroin, which method comprises the steps of:

    (i) preparing a mixture comprising fibrinogen and a silk fibroin solution in an aqueous solution;
    (ii) adding thrombin to the mixture prepared in (i) and incubating under conditions allowing fibrinogen to convert into fibrin fibers that at least partially crosslink;
    (iii) contacting the reaction mixture obtained in (ii) with an alcohol and incubating under conditions allowing the IPN to form.

6.  The method according to claim 5, wherein the alcohol is selected from the group consisting of methanol, ethanol, any linear or branched ($C_3$-$C_{12}$-alkyl) alcohol, and a mixture thereof, wherein the alcohol is preferably ethanol.

7.  The method according to claim 5 or 6, wherein the alcohol is diluted in water, with concentration between 5 and 100% (*v/v*), preferably between 20 and 60% (*v/v*), more preferably wherein the concentration of the alcohol is 40 % (*v/v*).

8.  The method according to any of claims 5 to 7, wherein a drug is incorporated during steps (i), (ii) or (iii).

9.  The method according to any of claims 1 to 8, wherein the concentration of fibrinogen is between 2 and 20 mg/mL, preferably between 3 and 12 mg/mL, more preferably 10 mg/mL; and the concentration between silk fibroin is between 2 and 100 mg/mL, preferably between 5 and 60, more preferably 20 mg/mL.

10. The method according to any of claims 1 to 9, wherein the aqueous solution comprises a divalent salt, preferably calcium chloride, and optionally further comprises a second salt, which is preferably sodium chloride, and wherein the concentration of the divalent salt is between 1 and 50 mM, preferably 20 mM.

11. The method according to claim 10, wherein the concentration of the second salt is between 1 and 200 mM, preferably 50 mM.

12. A gel material comprising an interpenetrating polymer network (IPN) of fibrin and silk fibroin, obtainable by the method as defined in any of claims 1 to 11.

13. The gel material of claim 12, which further comprises a drug, for use as a material for drug delivery.

14. The gel material as defined in claim 12 or 13, for use as a material for tissue engineering, preferably skin tissue engineering.

15. The gel material as defined in claim 12 or 13, for use as a filling material.

**Figure 1**

**Figure 2**

**Figure 3**

**Figure 4**

Figure 5

Figure 6

Figure 7

Figure 8

Figure 9

Figure 10

**Figure 11**

**Figure 12**

**Figure 13**

Figure 14

Figure 15

Figure 16

Figure 17

*4 hours, 37°C*

| Mixture of SF 2%, Fbg 1% In pure water, NaCl 50 mM, CaCl₂ 20 mM | → | Addition of Tb 0.4 U/mL, HRP 100 U/mL and H₂O₂ 0.01% | → | SF/Fb 2/1 IPN |

Fibrinogen    Silk Fibroin    Thrombin    Horseradish peroxidase

**Figure 18**

*4 hours, 37°C*        *3 hours, 37°C*

| Mixture of SF 2%, Fbg 1% In pure water, NaCl 50 mM, CaCl₂ 20 mM | → | Tb 0.4 U/mL | → | SF/Fb 2/1 semi-IPN | → | Ethanol 40% | → | SF/Fb 2/1 IPN |

Fibrinogen    Silk Fibroin    Thrombin

**Figure 19**

Europäisches
Patentamt

European
Patent Office

Office européen
des brevets

# EUROPEAN SEARCH REPORT

Application Number

EP 17 30 6789

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2012/288564 A1 (KURISAWA MOTOICHI [SG] ET AL) 15 November 2012 (2012-11-15) * 11, 12, 60, 127; claims 1-4 * ----- | 1-4, 12-15 | INV. A61L27/26 A61L27/52 A61L27/54 A61L27/60 |
| Y | CN 106 009 709 A (NANTONG TEXTILE & SILK IND TECH RES INST) 12 October 2016 (2016-10-12) * reference is made to the enclosed translation; p.1, section technical field; claims 1-3 * ----- | 1-4, 12-15 | |
| X | WO 2015/048344 A2 (UNIV TUFTS [US]) 2 April 2015 (2015-04-02) * 27-30, 32, 47, 134, 225; claims 1-22 * ----- | 5-15 | |
| A | KUNDU BANANI ET AL: "Silk fibroin biomaterials for tissue regenerations", ADVANCED DRUG DELIVERY REVIEWS, vol. 65, no. 4, 5 November 2012 (2012-11-05), pages 457-470, XP028593336, ISSN: 0169-409X, DOI: 10.1016/J.ADDR.2012.09.043 * abstract * ----- | 1-15 | |

TECHNICAL FIELDS SEARCHED (IPC)

A61L

The present search report has been drawn up for all claims

| Place of search | Date of completion of the search | Examiner |
|---|---|---|
| The Hague | 13 June 2018 | Cadamuro, Sergio |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 3 498 309 A1**

## ANNEX TO THE EUROPEAN SEARCH REPORT
## ON EUROPEAN PATENT APPLICATION NO.

EP 17 30 6789

13-06-2018

| Patent document cited in search report | | | Publication date | Patent family member(s) | | | Publication date |
|---|---|---|---|---|---|---|---|
| US 2012288564 | A1 | | 15-11-2012 | NONE | | | |
| CN 106009709 | A | | 12-10-2016 | NONE | | | |
| WO 2015048344 | A2 | | 02-04-2015 | US | 2016235889 | A1 | 18-08-2016 |
| | | | | WO | 2015048344 | A2 | 02-04-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010058132 A

- US 9512279 B

**Non-patent literature cited in the description**

- **PALLOTTA et al.** *Biomaterials,* 2014, vol. 35, 3678-3687

- **KIM et al.** *Biomaterials,* 2005, vol. 26, 2775-2785